# EUROPÄISCHE PATENTSCHRIFT

(11) **EP 3 131 395 B1**
(45) Veröffentlichungstag und Bekanntmachung des Hinweises auf die Patenterteilung: **18.07.2018**
(21) Anmeldenummer: 15724504.4
(22) Anmeldetag: 08.04.2015
(51) Int. Cl.: A01N 37/36, A01N 37/44, C11D 1/62, C11D 3/39, C11D 3/395, C11D 11/00, A01N 25/30, A01N 59/00, A01N 59/08, A01N 33/12, C11D 3/20, A01N 47/44

(54) **VERFAHREN ZUR BEKÄMPFUNG VON SCHIMMEL, ALGEN SOWIE ANDEREN MIKROOGRANISMEN AUF UNTERSCHIEDLICHEN VON DIESEN ORGANISMEN BEFALLENEN WAND-, BODEN- ODER DECKENFLÄCHEN SOWIE MAUERWERK IM ALLGEMEINEN**
METHOD FOR FIGHTING MOLD, ALGAE, AND OTHER MICROORGANISMS ON VARIOUS WALL, FLOOR, OR CEILING SURFACES INFESTED WITH SAID ORGANISMS, AND ON MASONRY IN GENERAL
PROCÉDÉ POUR LUTTER CONTRE LES MOISISSURES, LES ALGUES AINSI QUE D'AUTRES MICRO-ORGANISMES SUR DIFFÉRENTES SURFACES DE PAROI, DE PLANCHER OU DE PLAFOND AINSI QUE SUR DES MAÇONNERIES EN GÉNÉRAL, ATTAQUÉES PAR CES ORGANISMES

(30) Priorität: 15.04.2014 AT 502802014
(43) Veröffentlichungstag der Anmeldung: 22.02.2017
(73) Patentinhaber: BMB Gebäudehygiene GmbH, 3552 Dross (AT)
(72) Erfinder: BRANDNER, Gerhard, A-3552 Dross (AT)
(74) Vertreter: Speringer, Markus
(86) Internationale Anmeldenummer: PCT/AT2015/050090
(87) Internationale Veröffentlichungsnummer: WO 2015/157786

(56) Entgegenhaltungen:
- EP-A1- 1 266 571
- WO-A1-2005/075350
- WO-A1-2008/116509
- JP-A- 2012 250 959
- US-A1- 2005 282 722

## Beschreibung

### Technisches Gebiet

Die Erfindung betrifft ein Verfahren zur Bekämpfung von Schimmel, Algen sowie anderen Mikroorganismen auf unterschiedlichen von diesen Organismen befallenen Wand-, Boden- oder Deckenflächen sowie auf Mauerwerk im Allgemeinen.

### Stand der Technik

Wandschimmel ist ein weit verbreitetes Übel im Wohn- und Arbeitsbereich von Gebäuden, jedoch auch in Lagerräumen, Kellern oder auf Außenfassaden, Terrassen und dergleichen. Zahlreiche Verfahren auf Basis von Säuren, Alkoholen, Oxidationsmitteln und Laugen geben kurzfristige Erfolge, aber nach wenigen Monaten kommt es häufig wieder zu Schimmelbefall, oder aber die angewandten Mittel sind hochaggressiv, wodurch ihre Anwendung, besonders im Wohnbereich, nur eingeschränkt möglich ist oder mit gewissen weiteren Gesundheitsrisiken aufgrund der verwendeten Chemikalien verbunden ist.

Im Allgemeinen werden bei den im Stand der Technik verwendeten Behandlungsmethoden einzelne Substanzen in Lösung oder auch Gemische verschiedener Substanzen auf die betroffenen Flächen aufgebracht. Einzelne Substanzen alleine sind jedoch oft nur wenig effektiv und Gemische entfalten ihre volle Wirkung oftmals bereits beim Zusammenmischen im jeweiligen Gefäß durch Reaktion der einzelnen Substanzen untereinander und wirken damit nicht mehr ausreichend stark an den betroffenen Stellen auf oder im jeweiligen Mauerwerk.

US2005/0282722 offenbart eine Reinigungslösung, die aus zwei unterschiedlichen Lösungen zusammengesetzt ist, nämlich aus einer ersten Lösung bestehend aus mehreren Tensidverbindungen und einer zweiten Lösung bestehend aus Natriumhypochlorit und Natronlauge. Die beiden Lösungen, die insbesondere zur Entfernung von Seifen und Schimmel auf festen Oberflächen eingesetzt werden, werden vor der Anwendung gemischt.

EP1266 571 beschreibt Desinfektionslösungen bestehend aus Wasserstoffperoxid, Milchsäure, einem Biguanid, einem Schwermetallsalz und einer quartären Ammoniumverbindung. Die Lösung wird verwendet zur Desinfektion von Industrieeinrichtungen.

Außerdem ist allen im Stand der Technik bekannten Verfahren gemein, dass die aufgebrachten Lösungen lediglich zu einem oberflächlichen Abtöten und Desinfizieren der betroffenen Fläche führen. Ein vollständiges Entfernen des organischen Materials aus der betroffenen Fläche kann bislang nur durch Abstemmen der Wand erreicht werden, was mit deutlich höheren Kosten verbunden ist. Ein Wiederherstellen der betroffenen Fläche ohne kompletten Austausch des Untergrunds ist bisher praktisch unbekannt.

### Darstellung der Erfindung

Es ist somit Aufgabe der vorliegenden Erfindung die oben genannten Nachteile zu beseitigen und ein Verfahren zu schaffen, welches nachhaltig und effektiv von Schimmel, Algen und anderen Mikroorganismen befallene Wand-, Boden- und Deckenflächen befreit, dabei nicht nur oberflächliche Wirkung sondern auch Tiefenwirkung an den betroffenen Stellen zeigt, und welches mit Substanzen auskommt, die keine giftigen Rückstände oder Abbauprodukte hinterlassen, wodurch das Verfahren insbesondere auch in Wohnräumen oder Essbereichen angewendet werden kann.

Diese Aufgabe wird erfindungsgemäß durch ein Verfahren gelöst, dass die folgenden hintereinander ausgeführten Schritte umfasst:
-) Aufbringen einer ersten Lösung auf die befallene Fläche, wobei die erste Lösung mindestens eine Tensidverbindung enthält,
-) zumindest einmaliges Aufbringen einer durch Natronlauge basisch gemachten zweiten Lösung auf die befallene Fläche, wobei die Lösung zumindest eine Hypochloritverbindung enthält,
-) Aufbringen einer dritten Lösung auf die befallene Fläche, wobei die Lösung als aktive Substanzen zumindest Wasserstoffperoxid sowie Milchsäure enthält, und wobei das Aufbringen der dritten Lösung so lange fortgesetzt wird, bis die Schaumbildung auf der befallenen Fläche beendet ist
-) Aufbringen einer vierten Lösung auf die befallene Fläche, wobei die Lösung zumindest eine quartäre Ammoniumverbindung enthält.

Unter "Bekämpfung" wird im Rahmen dieser Anmeldung nicht allein das Abtöten der unerwünschten Organismen verstanden, sondern auch das Aufbrechen etwaiger Biofilme sowie das möglichst vollständige Entfernen des organischen Materials aus der betroffenen, meist porösen, Fläche.

Für den ersten Verfahrensschritt wird eine erste Lösung verwendet, welche zumindest eine Tensidverbindung beinhaltet. Als Tensidverbindungen kommen unter anderem Substanzen der folgenden Gruppen in Frage: lineare Alkylbenzolsulfonate, Alkylpolyglycoside, Esterquats, Fettalkoholethoxylate, Fettalkoholsulfate, Fettalkoholethersulfate.

Die erste Lösung sollte im Wesentlichen im neutralen oder vorzugsweise leicht alkalischen pH-Bereich angesiedelt sein (pH von 6-8). Die Wirkung der ersten Lösung besteht darin, dass die Poren der befallenen Fläche "geöffnet" werden und die Kapillarwirkung der Fläche verstärkt wird, wodurch das Schimmelmycel in den weiteren Verfahrensschritten auch bis in die Tiefe der jeweiligen Fläche hinein mit den nachfolgenden Lösungen benetzt werden kann. Da es sich bei von Schimmel befallenen Mauerflächen ohnehin oft um Flächen mit einer bestehenden Mauerfeuchte handelt, wird in jenen Fällen die ohnehin schon vorhandene Kapillarwirkung der Mauer durch die Zugabe der Tensidlösung weiter verstärkt und die jeweilige Fläche beginnt sozusagen zu "saugen". Es ist notwendig diesen Schritt vor dem Aufbringen der zweiten Lösung durchzuführen, um eine erhöhte Tiefenwirkung zu erreichen und ein Eindringen der nachfolgend aufgebrachten zweiten Lösung bis zu einer Tiefe von mehreren Zentimetern, je nach Untergrund, zu ermöglichen.

Die anschließend zweite aufgebrachte Lösung wird nach dem Aufbringen in die Wand gezogen, wirkt als Biozid und zerstört so auch mit entsprechender Tiefenwirkung Mikroorganismen und organisches Material. Außerdem führt die zweite Lösung zu einer Kaltbleichung der schwarzen oder grünen Verfärbungen, welche durch den Schimmel bzw. durch Algen verursacht sind. Das eingebrachte Hypochlorid wird in diesem Schritt aufgrund des erhöhten pH-Werts der vorbereiteten Fläche teilweise zu Chlorit, Chlorat und Perchlorat umgewandelt. Der zweite Verfahrensschritt kann je nach Fläche gegebenenfalls mehrfach wiederholt werden.

Beim anschließenden Aufbringen der dritten Lösung kommt es zu mehreren chemischen Reaktionen im Mauerwerk. Bei Zusammentreffen der Milchsäure mit dem aktiven Restchlor aus der zweiten Lösung, welche zuvor aufgebracht wurde, werden minimale Mengen Chlorgas freigesetzt. Diese Reaktion steht in Konkurrenz mit den weiteren chemischen Reaktionen zwischen dem Restchlor und dem Wasserstoffperoxid. Im feuchten Wandmilieu reagieren diese zuerst zu Chlorwasserstoff und anschließend zu wässriger Salzsäure. Diese reagiert mit der ebenfalls in der Wand befindlichen Natronlauge zu Wasser und Kochsalz. Durch die Reaktion zwischen Säure und Base, welche auch zwischen der eingebrachten Milchsäure mit der Natronlauge stattfindet, entsteht auch Hitze, welche thermisch auf die Mikroorganismen und Biofilme einwirkt. Das im zweiten Schritt gebildete Chlorit wird in Gegenwart der nun eingebrachten Säure ferner zu Chlordioxid umgewandelt und führt so zu einem wirksamen Aufbrechen der vorhandenen Biofilme. Die Reaktion des Hypochlorits mit dem Wasserstoffperoxid, welche zum Chlorwasserstoff führt, hat eine massive Ausdehnung zur Folge, welche das abgetötete und aufgebrochene organische Material chemomechanisch aus der Mauer drückt. Dieser Verfahrensschritt wird solange wiederholt, bis an der behandelten Fläche keine Schaumbildung mehr auftritt, das gesamte Chlor aus der zweiten Lösung folglich aufgebraucht ist.

In diesem Verfahrensschritt kommt es zu einer vollständigen Entfernung des gesamten organischen Materials inklusive der in der Wand verwurzelten Myzele. Damit sind auch Schimmelflecken oder Algenverfärbungen vollständig entfernt. Weiters neutralisiert dieser Verfahrensschritt alle Substanzen der vorhergehenden Schritte, treibt auch die Restsubstanzen der vorhergehenden Lösungen aus der Wand und sichert somit zu, dass anschließend keine toxischen oder ätzenden Substanzen mehr in bzw. auf der jeweiligen Fläche verbleiben. Wenn sehr viel organisches Material aus der behandelten Fläche austritt, kann es vorkommen, dass die aufgebrachten Flüssigkeiten eine unzureichende Schwemmkraft entwickeln. In diesem Fall wäre ein mechanisches Abwischen bzw. Abwaschen als Zwischenschritt notwendig, um das organische Material vor dem nächsten Verfahrensschritt von der Oberfläche zu entfernen.

Die vierte Lösung wird anschließend aufgebracht, um die weit geöffneten Mauerporen zu verkleinern bzw. zu verschließen und damit eine Rekontamination von Neuschimmelbefall weitestgehend zu vermeiden. Außerdem dienen die in der vierten Lösung enthaltenden quartären Ammoniumverbindungen als toxikologisch unbedenkliches Desinfektionsmittel. Als mögliche quartäre Amoniumverbindungen für die Verwendung in der vierten Lösung kommen unter anderem folgende Verbindungsgruppen in Frage: Alkyltrimethylammoniumsalze, Dialkyldimethylammoniumsalze, Benzalkoniumsalze, Esterquats, ethoxylierte quartäre Ammoniumverbindungen, alkaloide Sanguinarine.

Es ist ein weiteres Merkmal der Erfindung, dass die erste Lösung zumindest Natrium-Alkansulfonat mit einer Konzentration von 1-5%, Alkoholexthoxylat mit einer Konzentration von 1-5%, Butyldiglykol mit einer Konzentration von 5-15% und Pentakaliumtriphosphat mit einer Konzentration von 1-5% in Wasser enthält. Die Beimischung von Alkohol in der ersten Lösung bewirkt ein Schrumpfen der Myzelfäden, wodurch die Poren noch weiter geöffnet werden. Bei alle in der Beschreibung und in den Ansprüchen genannten Prozentangaben handelt es sich um Angaben in Gewichtsprozent.

Gemäß einem weiteren Merkmal der Erfindung ist es vorgesehen, dass die zweite Lösung zumindest Natriumhydroxid mit einer Konzentration von 5-15% und Natriumhypochlorit mit einer Konzentration von 1-10% in Wasser enthält. Anstelle von Hypochlorit können natürlich in analoger Weise auch andere geeignete Halogenidverbindungen eingesetzt werden. Bei vielen bekannten Verfahren findet lediglich eine Behandlung mit Hypochlorit statt, wodurch zwar oberflächlich der Schimmel beseitigt wird und eine Kaltbleiche stattfindet, jedoch die im Mauerwerk sitzenden tieferliegenden Myzelfäden nicht erreicht werden, weshalb ein rasches Nachwachsen des Schimmels möglich ist. Im vorliegenden Fall kann die zweite Lösung tief eindringen und somit den Befall vollständig zerstören. Je nach Material der befallenen Fläche und der damit verbundenen Eindringtiefe kann die zweite Lösung auch mehrfach aufgebracht werden.

Ferner ist es ein Merkmal der Erfindung, dass die dritte Lösung zumindest Wasserstoffperoxid mit einer Konzentration von 1-5%, Polyaminopropylbiguanid mit einer Konzentration von bis zu 1% und Milchsäure mit einer Konzentration von 1-5% in Wasser enthält. Anstelle von Milchsäure können natürlich auch andere Säuren eingesetzt werden. Bei diesem Reaktionsschritt entsteht auf der behandelnden Fläche eine heftige Schaumbildung, wobei die Behandlung so lange fortgesetzt wird, bis diese Schaumbildung beendet ist. Alles abgetötete organische Material wird aus den Mauerporen ausgeschwemmt und der pH-Wert im Mauerwerk wieder neutralisiert.

Es ist ein weiteres Merkmal der Erfindung, dass die vierte Lösung zumindest Benzalkoniumchlorid mit einer Konzentration von 5-15% und Ethylendiamintetraacetat mit einer Konzentration von bis zu 1% in Wasser enthält. Die vierte Lösung dient zum Versiegeln der behandelten Fläche und weist eine hohe Persistenz im Mauerwerk auf, kann aber im Boden biologisch leicht abgebaut werden. Sie ist auch für Menschen unschädlich und kann daher auch in Wohn- und Essbereichen unbedenklich eingesetzt werden.

Gemäß einem weiteren Merkmal der Erfindung ist es vorgesehen, dass anschließend an den letzten Verfahrensschritt bei einer Anwendung des Verfahrens auf Flächen in einem Innenraum die dritte Lösung in der Raumluft vernebelt wird. Die Verneblung kann in Innenräumen eine abschließende Desinfektion der Umgebung darstellen, wobei die Substanzen der dritten Lösung ebenfalls einfach abgebaut werden bzw. nach kurzer Zeit zerfallen und keine Gefahr für den Menschen darstellen.

Schließlich ist es ein weiteres Merkmal der Erfindung, dass das Aufbringen der jeweiligen Lösungen durch Aufsprühen erfolgt. Prinzipiell können die Lösungen durch unterschiedliche Methoden auf die betroffenen Flächen aufgebracht werden, wie Aufstreichen, Besprühen, Übergießen, etc. Als bevorzugte Methode werden die jeweiligen Lösungen jedoch gesprüht, da hier die eingesetzte Menge der verwendeten Lösungen sehr gering ist und ein gutes und gleichmäßiges Auftragen auf den betroffenen Stellen ermöglicht wird.

Das erfindungsgemäße Verfahren eignet sich auch für kunststoffgebundene Außenputze, wie sie beispielsweise an den Außenwänden von wärmegedämmten Häusern verwendet werden. Die Behandlung kann dabei auch nach dem dritten Verfahrensschritt beendet werden, weil der Kunststoff anders reagiert und die Poren nicht weiter nachbehandelt werden müssen. In diesem Fall reicht ein Abspülen der Wand mit Wasser, um die biologischen Reste des Bewuchses wieder zu entfernen.

### Weg(e) zur Ausführung der Erfindung

Im nachfolgenden werden ein Ausführungsbeispiel für das erfindungsgemäße Verfahren sowie die darin verwendeten Lösungen angegeben.

Eine mit Wandschimmel befallene Wand mit ca. 5 m² wurde mit dem erfindungsgemäßen Verfahren mit den unten angegebenen Lösungen behandelt.

Die erste Lösung mit einer eingesetzten Menge von 250 ml wurde aufgesprüht.

11 der ersten Lösung enthalten:
Natrium-Alkansulfonat - 125 g
Alkoholexthoxylat - 125 g
Butyldiglykol - 375 g
Pentakaliumtriphosphat - 125 g

Nach einer Einwirkzeit von 15 min wurde die zweite Lösung aufgebracht. Aufgrund des Mauerwerks (Vollziegelwand in einem Altbau, verputzt, weiß gestrichen) wurde die zweite Lösung 3 Mal aufgesprüht. Insgesamt wurde eine Menge von 21 verwendet.

11 der zweiten Lösung enthalten:
Natriumhydroxid - 300 g
Natriumhypochlorit - 200 g

Nach einer Einwirkzeit von 30 min wurde die dritte Lösung 2 mal aufgesprüht. Insgesamt wurde eine Menge von 21 verwendet.

11 der dritten Lösung enthalten:
Wasserstoffperoxid - 100 g
Polyaminopropylbiguanid - 10 g
Milchsäure - 100 g/ml

Nach der letzten Behandlung konnte keinerlei Schaumbildung mehr wahrgenommen werden. Schließlich wurde die vierte Lösung aufgesprüht. Insgesamt wurde eine Menge von 500 ml verwendet.

11 der vierten Lösung enthalten:
Benzalkoniumchlorid - 0,75 g
Ethylendiamintetraacetat - 0,05 g

Abschließend wurden 250 ml der dritten Lösung in einem Vernebler (N80101240 PfalzTechnik AUTOMATIC W03, S-B10.W03 DD; Durchsatz 2,5-3 bar: 2-7 l/min effektiv; erzeugte Aerosolteilchen haben eine Größe von ca. 8 µm) für 5 min im Raum, in dem sich die behandelte Fläche befindet, vernebelt. Nach ca. 160 min war das Verfahren abgeschlossen und der Raum konnte gefahrlos benutzt werden.

Da die Wand vorher durch einen Kasten verstellt war, war nur eine mangelnde Hinterlüftung als schimmelauslösend anzusehen. Da die Wand trocken ist und der Baukörper drainagiert ist, ist von einer Neuanschimmelung nicht vor 3 Jahren auszugehen.

## Patentansprüche

1. Verfahren zur Bekämpfung von Schimmel, Algen sowie anderen Mikroorganismen auf unterschiedlichen von diesen Organismen befallenen Wand-, Boden- oder Deckenflächen sowie auf Mauerwerk im Allgemeinen, **dadurch gekennzeichnet, dass** das Verfahren die folgenden hintereinander ausgeführten Schritte umfasst:
-) Aufbringen einer ersten Lösung auf die befallene Fläche, wobei die erste Lösung mindestens eine Tensidverbindung enthält,
-) zumindest einmaliges Aufbringen einer durch Natronlauge basisch gemachten zweiten Lösung auf die befallene Fläche, wobei die Lösung zumindest eine Hypochloritverbindung enthält,
-) Aufbringen einer dritten Lösung auf die befallene Fläche, wobei die Lösung als aktive Substanzen zumindest Wasserstoffperoxid sowie Milchsäure enthält, und wobei das Aufbringen der dritten Lösung so lange fortgesetzt wird, bis die Schaumbildung auf der befallenen Fläche beendet ist
-) Aufbringen einer vierten Lösung auf die befallene Fläche, wobei die Lösung zumindest eine quartäre Ammoniumverbindung enthält.

2. Verfahren nach Anspruch 1, **dadurch gekennzeichnet, dass** die erste Lösung zumindest Natrium-Alkansulfonat mit einer Konzentration von 1-5%, Alkoholexthoxylat mit einer Konzentration von 1-5%, Butyldiglykol mit einer Konzentration von 5-15% und Pentakaliumtriphosphat mit einer Konzentration von 1-5% in Wasser enthält.

3. Verfahren nach Anspruch 1 oder 2, **dadurch gekennzeichnet, dass** die zweite Lösung zumindest Natriumhydroxid mit einer Konzentration von 5-15% und Natriumhypochlorit mit einer Konzentration von 1-10% in Wasser enthält.

4. Verfahren nach einem der Ansprüche 1 bis 3, **dadurch gekennzeichnet, dass** die dritte Lösung zumindest Wasserstoffperoxid mit einer Konzentration von 1-5%, Polyaminopropylbiguanid mit einer Konzentration von bis zu 1% und Milchsäure mit einer Konzentration von 1-5% in Wasser enthält.

5. Verfahren nach einem der Ansprüche 1 bis 4, **dadurch gekennzeichnet, dass** die vierte Lösung zumindest Benzalkoniumchlorid mit einer Konzentration von 5-15% und Ethylendiamintetraacetat mit einer Konzentration von bis zu 1% in Wasser enthält.

6. Verfahren nach einem der Ansprüche 1 bis 5, **dadurch gekennzeichnet, dass** anschließend an den letzten Verfahrensschritt bei einer Anwendung des Verfahrens auf Flächen in einem Innenraum die dritte Lösung in der Raumluft vernebelt wird.

7. Verfahren nach einem der Ansprüche 1 bis 5, **dadurch gekennzeichnet, dass** das Aufbringen der jeweiligen Lösungen durch Aufsprühen erfolgt.

## Claims

1. A method of fighting mold, algae and other microorganisms on various wall, floor or ceiling surfaces infested therewith, and on masonry in general, **characterized in that** the method consists of the steps of sequentially:
applying to the affected surface a first solution that contains at least one surfactant compound,
applying to the affected surface a second solution that has been made alkaline using sodium hydroxide on the affected surface and that contains at least one hypochlorite compound,
applying to the affected surface a third solution that contains at least hydrogen peroxide and lactic acid as active substances and continuing application of the third solution until foaming on the affected surface has ended,
applying to the affected surface a fourth solution that contains at least one quaternary ammonium compound.

2. The method according to claim 1, **characterized in that** the first solution contains at least sodium alkane sulfonate with a concentration of 1-5%, alcohol ethoxylate with a concentration of 1-5%, butyldiglycol with a concentration of 5-15% and pentapotassium triphosphate with a concentration of 1-5%.

3. The method according to claim 1 or 2, **characterized in that** the second solution is aqueous and contains at least sodium hydroxide with a concentration of 5-15% and sodium hypochlorite with a concentration of 1-10%.

4. The method according to one of the claims 1 to 3, **characterized in that** the third solution is aqueous and contains at least hydrogen peroxide with a concentration of 1-5%, polyaminopropyl biguanide with a concentration of up to 1% and lactic acid with a concentration of 1-5%.

5. The method according to one of the claims 1 to 4, **characterized in that** the fourth solution is aqueous and contains at least benzalkonium chloride with a concentration of 5-15% and ethylenediaminetetraacetate with a concentration of up to 1%.

6. The method according to one of the claims 1 to 5, **characterized in that**, subsequent to the final step of the method when it is used on surfaces in an internal room, the third solution is nebulized in the air of the room.

7. The method according to one of the claims 1 to 5, **characterized in that** each of the solutions is applied by spraying onto the surface.

## Revendications

1. Procédé de lutte contre les moisissures, les algues ainsi que d'autres micro-organismes sur différentes surfaces de mur, de sol ou de plafond atteintes desdits organismes, ainsi que sur des ouvrages de maçonnerie en général, **caractérisé en ce que** ledit procédé comprend les étapes successives suivantes :
-) application d'une première solution sur la surface atteinte, la première solution contenant au moins un composé tensioactif,
-) application, au moins une fois, d'une deuxième solution sur la surface atteinte, ladite solution présentant un caractère basique suite à l'ajout d'une préparation aqueuse de soude caustique tout en contenant au moins un composé d'hypochlorite,
-) application d'une troisième solution sur la surface atteinte, ladite solution contenant en tant que substances actives au moins du peroxyde d'hydrogène ainsi que de l'acide lactique, ladite application de la troisième solution étant continuée jusqu'à ce que plus aucune mousse ne se forme sur la surface atteinte
-) application d'une quatrième solution sur la surface atteinte, ladite solution contenant au moins un composé d'ammonium quaternaire.

2. Procédé selon la revendication 1, **caractérisé en ce que** la première solution contient au moins de l'alcanesulfonate de sodium à une concentration de 1 à 5 %, de l'éthoxylate d'alcool à une concentration de 1 à 5 %, du butyldiglycol à une concentration de 5 à 15 % et du triphosphate de pentapotassium à une concentration de 1 à 5 % dans de l'eau.

3. Procédé selon les revendications 1 ou 2, **caractérisé en ce que** la deuxième solution contient au moins de l'hydroxyde de sodium à une concentration de 5 à 15 % et de l'hypochlorite de sodium à une concentration de 1 à 10 % dans de l'eau.

4. Procédé selon l'une des revendications 1 à 3, **caractérisé en ce que** la troisième solution contient au moins du peroxyde d'hydrogène à une concentration de 1 à 5 %, du biguanide de polyaminopropyle à une concentration inférieure ou égale à 1 % et de l'acide lactique à une concentration de 1 à 5 % dans de l'eau.

5. Procédé selon l'une des revendications 1 à 4, **caractérisé en ce que** la quatrième solution contient au moins du chlorure de benzalkonium à une concentration de 5 à 15 % et de l'éthylène-diamine-tétraacétate à une concentration inférieure ou égale à 1 % dans de l'eau.

6. Procédé selon l'une des revendications 1 à 5, **caractérisé en ce que**, suite à la dernière étape de procédé dans le cadre d'une mise en oeuvre dudit procédé sur des surfaces situées à l'intérieur d'une pièce, on pulvérise la troisième solution dans l'air de ladite pièce.

7. Procédé selon l'une des revendications 1 à 5, **caractérisé en ce que** l'application de chacune desdites solutions est réalisée par pulvérisation ciblée.
